# EUROPEAN PATENT APPLICATION

(11) **EP 2 905 020 A1**
(43) Date of publication of application: **12.08.2015**
(21) Application number: 15153747.9
(22) Date of filing: 04.02.2015
(51) Int. Cl.: A61K 9/46, A61K 9/16, A61K 31/192, A61K 31/198

(54) **Effervescent formulations comprising ibuprofen and N-acetylcystein**

(30) Priority: 05.02.2014 TR 201401277
(71) Applicant: Sanovel Ilac Sanayi ve Ticaret A.S., 34460 Istanbul (TR)
(72) Inventor: Türkyilmaz, Ali, 34460 Istanbul (TR); Yildirim, Ediz, 34460 Istanbul (TR); Bas, Oguz, 34460 Istanbul (TR); Kartal, Burcu, 34460 Istanbul (TR)
(74) Representative: Sevinç, Erkan

(57) **Abstract**

The present invention relates to an effervescent formulation comprising ibuprofen or a pharmaceutically acceptable salt thereof and N-acetylcystein or a pharmaceutically acceptable salt thereof and one or more pharmaceutically acceptable excipients.

## Description

### Field of Invention

The present invention relates to an effervescent formulation comprising ibuprofen or a pharmaceutically acceptable salt thereof and N-acetylcystein or a pharmaceutically acceptable salt thereof and one or more pharmaceutically acceptable excipients.

### Background of Invention

Ibuprofen is known as a therapeutic agent having analgesic, anti-inflammatory and antipyretic activity and its chemical name is 2-(4-isobutylphenyl)propionic acid and its chemical structure is shown in the Formula I.

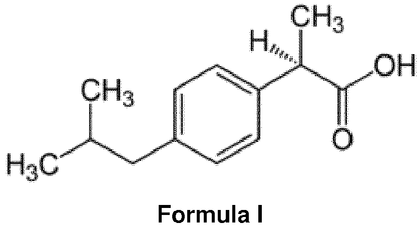

The patent application US3385886 (A) discloses the ibuprofen molecule. Ibroprofen reduces hormones leading to inflammation and pain in the body and used for reducing fever and treating pain or inflammation caused by many conditions such as headache, toothache, back pain, arthritis, menstrual cramps, or minor injury.

N-acetylcysteine is a derivative of cysteine and its chemical name is (2R)-2-acetamido-3-sulfanylpropanoic acid and its chemical structure is shown in the Formula II.

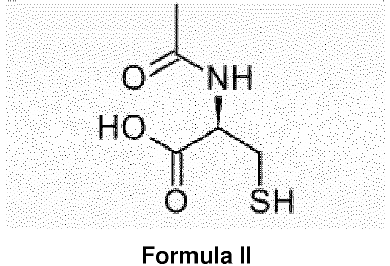

The patent application US3184505 (A) discloses the preparation process of the N-acetyl cysteine. N-acetyl cysteine is used primarily as a mucolytic agent for the treatment of respiratory diseases. It is used as a cough medicine because it breaks disulfide bonds in mucus and liquefies it, making it easier to cough up. Also this action of breaking disulfide bonds that makes it useful in thinning the abnormally thick mucus in cystic and pulmonary fibrosis patients.

The patent GB2192790 (B) discloses a water-soluble effervescent pharmaceutical composition comprising N-acetylcysteine, citric acid, sodium bicarbonate, aspartame and flavouring agent.

The patent application US4689218 (A) discloses a pharmaceutical composition consisting of Ibuprofen, arginine, sodium or potassium bicarbonate, sodium bitartrate in the form of an effervescent tablet or granulate.

The patent application EP2662077 (A1) discloses an effervescent composition containing N-acetylcysteine, a carbonate or bicarbonate salt and a mixture of a sweetener, a flavor, and a diluent.

In the state of art, there are several patents and applications which disclose effervescent formulations of ibuprofen or N-acetylcystein as mentioned above, but none of them comprising both ibuprofen and N-acetylcystein together in an effervescent formulation. In the present invention, an effervescent composition comprising both ibuprofen and N-acetylcystein is disclosed.

Effervescent tablets are designed to break down quickly and release carbon dioxide when dropped in liquid. They have gained considerable attention as a preferred alternative to conventional tablets and capsules due to their better patient compliance. Today, there are a growing number of people who cannot swallow tablets or capsules. In addition to ease of administration, many studies have demonstrated that effervescent formulations enhance absorption of a number of active ingredients and reduce the side effects of a drug. They generally contain acidifying agents and alkalizing agents which react rapidly in the presence of water by releasing carbon dioxide such as carbonates or bicarbonates. Carbon dioxide emitted by the effervescent reaction can induce enhanced active-ingredient permeability due to an alteration of the paracellular pathway. Moreover, the low pH in the stomach can cause active ingredients to become denatured, lose activity, or cause them to remain inactive. Acidifying agent - alkalizing agent couples, however, can buffer the water-active solution so that the stomach pH increases and thus prevent the degradation or inactivation of the active ingredient.

On the other side, effervescent formulations are not easy to process. Effervescent tablets should ensure a fast solubilization of the drug, to provide neutral, clear solution without any residue and precipitation and to have a pleasant taste. Therefore, a need rises in developing an effervescent formulation which has a desired disintegration time and favorable solubility without any residue and precipitation after tablet disintegration and acceptable taste for patients. Solubility problems of the drug and the formulation, unpleasant and bitter taste upon dissolution in water are problems that should be overcome.

### Detailed Description of Invention

The present invention relates to an effervescent formulation comprising ibuprofen or a pharmaceutically acceptable salt thereof and N-acetylcystein or a pharmaceutically acceptable salt thereof and one or more pharmaceutically acceptable excipients.

The objects of this invention are to overcome above mentioned problems related to effervescent formulations like obtaining desirable solubility of the drug and formulation without any residue or precipitation after disintegration and better taste for patients by using appropriate excipients together and using the suitable excipients in specific ratios.

The basic difficulties encountered when two or more molecules are combined in the same pharmaceutical dosage form are (a) ensuring compatibility between different active agents and/or among the active agents and the excipients used, (b) providing therapeutic compatibility between the active agents, taking into account the pharmacokinetic and/or biopharmaceutical properties such as the posology of the respective combination to obtain efficient and reliable plasma levels of both active agents. Therefore the selection of the active agents used together and other appropriate excipients and the amounts used are crucial.

According to one embodiment, ibuprofen or a pharmaceutically acceptable salt thereof is present in an amount of 1 to 20 %, preferably 2 to 15 % and more preferably 4 to 10% by weight of total formulation and N-acetylcystein or a pharmaceutically acceptable salt thereof is present in an amount of 3 to 50 %, preferably 5 to 40 % and more preferably 10 to 30 % by weight of total formulation.

The pharmaceutical formulations according to the present invention may also comprise one or more pharmaceutically acceptable excipient(s). Pharmaceutically acceptable excipients comprise, but are not limited to, acidifying agents, alkalizing agents, binders, lubricants, sweeteners, aromatic agents, or the mixtures thereof.

In general, effervescent formulations comprise acidifying agents and alkalizing agents such as carbonates or bicarbonates which react rapidly in the presence of water by releasing carbon dioxide. Choice of acidifying agent - alkalizing agent couple and their ratio is important in terms of stability, disintegration time and water solubility.

In order to get a neutral solution without any residue and precipitation after tablet disintegration, the ratio of acidifying agent to alkalizing agent should be determined carefully. Gastrointestinal system may be effected negatively due to high amount of acidifying agent. Also high amount of alkalizing agent (especially hydrophobic agents) leads to unclear and precipitated solution and damages in gastrointestinal system. In this invention, within a specific ratio of them, desired neutral solution without any precipitation has been achieved.

Moreover, since ibuprofen has a bitter taste and can be irritant for the mucosa of the mouth, it creates undesirable taste for patients, but it has been surprisingly found that the use of suitable acidifying agent - alkalizing agent couple in specific ratios with ibuprofen makes considerable contribution to obtain pleasent taste besides sweeteners especially sucrose and shaccarin sodium and aromatic agents used in the present formulation by yielding neutral clear solution without any residue and precipitation.

According to this embodiment, the ratio of the acidifying agents to the alkalizing agents is between 700:1 (w/w) and 1:250 (w/w), preferably between 200:1 (w/w) and 1:150 (w/w) and more preferably it is between 100:1 (w/w) and 1.100 (w/w).

According to this embodiment, the acidifying agent is selected from the group comprising malic acid, fumaric acid, citric acid, adipic acid, acetic acid, hydrochloric acid, nitric acid, phosphoric acid, sulfuric acid, tartaric acid or mixtures thereof, preferably it is malic acid.

According to this embodiment, the amount of malic acid is in the range of 0.1 to 70 %, preferably 0.2 to 50 %, more preferably it is 0.3 to 40 % by weight of total formulation. According to this embodiment, the alkalizing agent is selected from the group comprising sodium bicarbonate, sodium glycine carbonate, ammonia solution, ammonium carbonate, diethanolamine, diisopropanolamine, potassium hydroxide, sodium borate, sodium carbonate, sodium hydroxide, trolamine or mixtures thereof, preferably it is sodium bicarbonate.

According to this embodiment, the amount of sodium bicarbonate is in the range of 0.1 to 83 %, preferably 0.3 to 50 %, more preferably it is 0.5 to 40 % by weight of total formulation.

According to this embodiment, ibuprofen has a low water solubility and its salts also require long time and heating for solubilization in water, and it causes gastric irritation when administered in solid or suspended form. Therefore, it is needed to overcome the solubility problem of ibuprofen in order to provide administration of solubilized form of ibuprofen. Accordingly, in the present invention, it has been surprisingly found that the water solubility of the ibuprofen is improved by using sodium bicarbonate. Sodium bicarbonate facilitates to solubilization of ibuprofen and provides to eliminate the mucosa of the mouth and gastric irritation. Another significance of using sodium bicarbonate with ibuprofen is its taste-masking property. It masks the undesirable taste of ibuprofen and provides pleasant taste for patients.

In a preferred embodiment of the present invention, said binders comprise, but are not limited to, polyvinylpyrrolidone (povidone), low-substituted hydroxypropyl cellulose, xanthan gum, gelatin, sugars, glucose, natural gums, gums, synthetic celluloses, polymethacrylate, hydroxypropyl methylcellulose, hydroxypropyl cellulose, carboxymethyl cellulose, methyl cellulose, other cellulose derivatives, or the mixtures thereof, preferably, it is polyvinylpyrrolidone (povidone).

According to this embodiment, the amount of polyvinylpyrrolidone (povidone) is in the range of 1 to 10 %, preferably 2 to 8 %, more preferably it is 3 to 7 % by weight of total formulation.

In a preferred embodiment of the present invention, said lubricants comprise, but are not limited to sodium lauryl sulphate, sodium stearyl fumarate, magnesium stearate, polyethylene glycol, metal stearates, hydrogenated castor oil or the mixtures thereof, preferably, it is sodium lauryl sulphate.

According to this embodiment, the amount of sodium lauryl sulphate is in the range of 0.1 to 10 %, preferably 0.5 to 5 %, more preferably it is 1 to 4 % by weight of total formulation.

In a preferred embodiment of the present invention, said sweeteners comprise, but are not limited to, shaccarin sodium, sucrose, aspartame, sucralose, glucose, lactose, fructose and other sugars, or mannitol, sorbitol, xylitol, erythritol and other sugar alcohols or the mixtures thereof. Preferably, they are shaccarin sodium and sucrose.

According to this embodiment, the amount of shaccarin sodium is in the range of 5 to 85 %, preferably 10 to 80 %, more preferably it is 15 to 75 % by weight of total formulation.

According to this embodiment, the amount of sucrose is in the range of 5 to 85 %, preferably 10 to 80 %, more preferably it is 15 to 75 % by weight of total formulation.

In a preferred embodiment of the present invention, said aromatic agents comprise, but are not limited to fruit aromas such as orange, banana, strawberry, cherry, wild cherry, lemon, etc., and other aromas such as cardamom, anis, mint, menthol, vanillin or the mixtures thereof.

According to this embodiment, the amount of the aromatic agent is in the range of 0.1 to 5 %, preferably 0.5 to 4 %, more preferably it is 1 to 3 % by weight of total formulation.

The formulation according to the present invention may be in the form of an effervescent tablet or a sachet.

The present invention is used for reducing the symptomatic effects of acut chronic infections.

In this present invention, the formulation has been designed as following:
a) 1 to 20 % by weight of Ibuprofen
b) 3 to 50 % by weight of N-acetylcystein
c) 0.1 to 70 % by weight of malic acid
d) 0.1 to 83 % by weight of sodium bicarbonate
e) 1 to 10 % by weight of povidone
f) 0.1 to 10 % by weight sodium lauryl sulfate
g) 5 to 85 % by weight of shaccarin sodium
h) 5 to 85 % by weight of sucrose
i) 0.1 to 5 % by weight of aromatic agent

The example is not intended to limit the scope of the present invention, it should be considered in the light of the description detailed above.

### Example 1

| **Formulation Content** | **Amount (%)** |
|---|---|
| Ibuprofen | 2 - 15 |
| N-acetylcystein | 5 - 40 |
| Malic acid | 0.2 - 50 |
| Sodium bicarbonate | 0.3 - 50 |
| Povidone | 2 - 8 |
| Sodium lauryl sulfate | 0.5 - 5 |
| Saccharin sodium | 10 - 80 |
| Sucrose | 10 - 80 |
| Aromatic agent | 0.5 - 4 |

The pharmaceutical formulation mentioned above is prepared as following:
Ibuprofen, N-Acetylcystein, sodium bicarbonate, saccharin sodium, sodium lauryl sulfate, sucrose and povidone are pre-mixed in high shear granulator for 10 minutes.

This mixture is granulated up to getting suitable granulation level, with the addition of sufficient amount of alcohol. Granules are pre-sieved by using 2 mm of sieve and dried.

Dried granules and malic acid and aromatic agent are sieved together by using 1 mm of sieve and mixed for 15 minutes. The mixture is filled to sachets.

## Claims

1. An effervescent formulation comprising a combination of ibuprofen or a pharmaceutically acceptable salt thereof and N-acetylcystein or a pharmaceutically acceptable salt thereof and one or more pharmaceutically acceptable excipients.

2. The effervescent formulation according to claim 1, wherein ibuprofen or a pharmaceutically acceptable salt thereof is present in an amount of 1 to 20 %, preferably 2 to 15 % and more preferably 4 to 10% by weight of total formulation and N-acetylcystein or a pharmaceutically acceptable salt thereof is present in an amount of 3 to 50 %, preferably 5 to 40 % and more preferably 10 to 30 % by weight of total formulation.

3. The effervescent formulation according to claim 1 and 2, wherein one or more pharmaceutically acceptable excipient is selected from the group comprising acidifying agent, alkalizing agent, binders lubricants, sweeteners and aromatic agents.

4. The effervescent formulation according to any of the preceding claims, the ratio of the acidifying agents to the alkalizing agents is between 700:1 (w/w) and 1:250 (w/w), preferably between 200:1 (w/w) and 1:150 (w/w) and more preferably it is between 100:1 (w/w) and 1:100 (w/w).

5. The effervescent formulation according to any of the preceding claims, wherein the acidifying agent is selected from the group comprising malic acid, fumaric acid, citric acid, adipic acid, acetic acid, hydrochloric acid, nitric acid, phosphoric acid, sulfuric acid, tartaric acid or mixtures thereof, preferably it is malic acid.

6. The effervescent formulation according to any of the preceding claims, wherein the amount of malic acid is in the range of 0.1 to 70 %, preferably 0.2 to 50 %, more preferably it is 0.3 to 40 % by weight of total formulation.

7. The effervescent formulation according to any of the preceding claims, wherein the alkalizing agent is selected from the group comprising sodium bicarbonate, sodium glycine carbonate, ammonia solution, ammonium carbonate, diethanolamine, diisopropanolamine, potassium hydroxide, sodium borate, sodium carbonate, sodium hydroxide, trolamine or mixtures thereof, preferably it is sodium bicarbonate.

8. The effervescent formulation according to any of the preceding claims, wherein the amount of sodium bicarbonate is in the range of 0.1 to 83 %, preferably 0.3 to 50 %, more preferably it is 0.5 to 40 % by weight of total formulation.

9. The effervescent formulation according to any of the preceding claims, wherein the binder is selected from the group comprising polyvinylpyrrolidone (povidone), low-substituted hydroxypropyl cellulose, xanthan gum, gelatin, sugars, glucose, natural gums, gums, synthetic celluloses, polymethacrylate, hydroxypropyl methylcellulose, hydroxypropyl cellulose, carboxymethyl cellulose, methyl cellulose, other cellulose derivatives, or the mixtures thereof, preferably, it is polyvinylpyrrolidone (povidone).

10. The effervescent formulation according to any of the preceding claims, wherein the amount of polyvinylpyrrolidone (povidone) is in the range of 1 to 10 %, preferably 2 to 8 %, more preferably it is 3 to 7 % by weight of total formulation.

11. The effervescent formulation according to any of the preceding claims, wherein the lubricant is selected from the group comprising sodium lauryl sulphate, sodium stearyl fumarate, magnesium stearate, polyethylene glycol, metal stearates, hydrogenated castor oil, or the mixtures thereof, preferably, it is sodium lauryl sulphate.

12. The effervescent formulation according to any of the preceding claims, wherein the amount of sodium lauryl sulphate is in the range of 0.1 to 10 %, preferably 0.5 to 5 %, more preferably it is 1 to 4 % by weight of total formulation.

13. The effervescent formulation according to any preceding claims comprising;
a) 1 to 20 % by weight of Ibuprofen
b) 3 to 50 % by weight of N-acetylcystein
c) 0.1 to 70 % by weight of malic acid
d) 0.1 to 83 % by weight of sodium bicarbonate
e) 1 to 10 % by weight of povidone
f) 0.1 to 10 % by weight sodium lauryl sulfate
g) 5 to 85 % by weight of shaccarin sodium
h) 5 to 85 % by weight of sucrose
i) 0.1 to 5 % by weight of aromatic agent
